# EUROPEAN PATENT APPLICATION

(11) **EP 0 879 599 A2**
(43) Date of publication of application: **25.11.1998**
(21) Application number: 98303814.2
(22) Date of filing: 14.05.1998
(51) Int. Cl.: A61K 31/52, A61K 31/565

(54) **A topical preparation for treatment of erectile dysfunction comprising theophylline or aminophylline, isosorbide dinitrate and co-dergocrine mesylate**

(30) Priority: 18.05.1997 EG 43997
(71) Applicant: Abdel-Wadood Gomaa, Adel, Prof. Dr., Assiut (EG)
(72) Inventor: Abdel-Wadood Gomaa, Adel, Prof. Dr., Assiut (EG)
(74) Representative: Baldock, Sharon Claire

(57) **Abstract**

The present invention relates to a novel composition for the use in the treatment of erectile dysfunction in man and in the topical treatment thereof which composition comprises a mixture ofthe following ingredients:
a- Theophylline or aminophylline
b- Isosorbide dinitrate
c- Co-dergocrine mesylate (dihydro argotoxine mesylate) along with normal carriers and catalysts.

## Description

The present invention relates to a novel preparation for use in treatment of erectile dysfunction in men. In particular it relates to a composition prepared for use, topically in the treatment of erectile dysfunction.

It is known that the modern types of therapy of erectile dysfunction are based on the intracorporeal injection of vascoactive agents in the penile spongy tissues.

In spite of the good results achieved by this method it has dangerous drawbacks, most important of which is that it might lead to fibrosis in the said spongy tissues, it might also bring about permanent and painful erection causing tissue laceration. Actually this method is practically improper; as the patient can not be given injections by himself in a right way whenever he wants to make love, (i.e. intercourse).

The present invention aims at overcoming these disadvantages through compositions for topical application with efficiency equal to that used in the injections of the prior art, but without causing the side effects of using those injections. Meanwhile the said compositions can be easily used by the patient.

The novel compositions are used easily and are active in the treatment of erectile dysfunction as they have positive effect on the male once they are absorbed through the skin or cutaneous tissues.

A property of the novel composition is that it contains active materials including theophylline, isosorbide dinitrate and co-degrocrine mesylate ( and Hydro argo toxin mesylate). The said composition can be prepared by mixing the ingredients together with inert carriers or assistant factors ( catalysts) used in the field. The composition could be formulated as a cream, gel or spray ( normal or under pressure).

it was found that the said composition has topical effect and central action, in the treatment of erectile dysfunction.

It is well known that co-dergocrine has the ability of closing alfa-receptors in the cavernous artery, which helps in the artery lengthening and increase of the blood flow to the cavernous and spongy tissue.

On the other hand, isosorbide dinitrate and theophylline cause relaxation of the spongy sinus which become full of blood and press the veins which, in turn, decrease the blood escape from the spongy tissue.

This effect is due to the presence of co-dergocrine which has the property of isodopamin that assists the erectile activation in the brain and in the transmitter nerve ends in the penile tissue.

In controlled examples, a study has been effected to determine the action and effectiveness of each ingredient separately, in comparison with those of the present composition

Table 1 shows the action of co-dergocrine on the increment of mean blood flow ( M/S) of carvernous artery 15 minutes after 4 doses of co-dergocrine mesylate in psychogenic patients.

**Table 1:**

| Mean blood velocity ( F/S) after co-dergocrine application | | | | |
|---|---|---|---|---|
| Trial No. | CO-DERGOCRINE DOSES | | | |
| | 0.04% | 0.05% | 0.06% | 0.07% |
| 1 | 0.05 | 0.12 | 0.16 | 0.16 |
| 2 | 0.07 | 0.09 | 0.17 | 0.18 |
| 3 | 0.04 | 0.09 | 0.15 | 0.17 |
| 4 | 0.06 | 0.08 | 0.13 | 0.12 |
| 5 | 0.08 | 0.11 | 0.15 | 0.15 |

This mean blood velocity has been measured by the use of two ingredients one of which can be applied in the present application. Tables 2 and 3 show the results.

**Table 2 :**

| Mean blood velocity ( M/S) after Aminophylline application | | | | |
|---|---|---|---|---|
| Trial No. | AMINOPHYLLINE DOSES | | | |
| | 1% | 2% | 3% | 4% |
| 1 | 0.03 | 0.1 | 0.08 | 0.1 |
| 1 | 0.02 | 0.08 | 0.07 | 0.07 |
| 3 | 0.05 | 0.07 | 0.1 | 0.09 |
| 4 | 0.01 | 0.08 | 0.09 | 0.08 |
| 5 | 0.04 | 0.06 | 0.06 | 0.07 |

**Table 3:**

| Mean blood velocity ( M/.S) after theophylline application | | | | |
|---|---|---|---|---|
| Trial No | THEOPHYLLINE DOSES | | | |
| | 1% | 2% | 3% | 4% |
| 1 | 0.07 | 0.12 | 0.13 | 0.12 |
| 2 | 0.06 | 0.14 | 0.11 | 0.13 |
| 3 | 0.03 | 0.09 | 0.1 | 0.09 |
| 4 | 0.05 | 0.13 | 0.13 | 0.13 |
| 5 | 0.04 | 0.09 | 0.1 | 0.12 |

In the meantime, the increment of mean blood flow velocity of cavernous artery has been measured using the third ingredient of the present composition. Table 4 shows the results.

**Table 4:**

| Mean blood velocity ( M/S) after the application of isosorbide dinitrate | | | | |
|---|---|---|---|---|
| Trial No. | DOSES OF ISOSORBIDE DINITRATE | | | |
| | 0.1% | 0.25% | 0.3% | 0.5% |
| 1 | 0.06 | 0.08 | 0.12 | 0.14 |
| 2 | 0.08 | 0.09 | 0.11 | 0.13 |
| 3 | 0.05 | 0.07 | 0.15 | 0.16 |
| 4 | 0.05 | 0.08 | 0.1 | 0.15 |
| 5 | 0.07 | 0.1 | 0.09 | 0.16 |

The following examples show some of the present invention preparations, as way of example only.

### Example 1

### Preparation A: Cream for topical application:

A cream was prepared for topical application from the following ingredients

| % Weight | Ingredient |
|---|---|
| 0.05% | Co-dergocrine mesylate |
| 0.25% | Isosorbide dinitrate |
| 3% | aminophylline |

along with inert carriers of that kind normally used in preparing the creams.

### Example 2:

### Preparation B: cream preparation for topical application

A cream was prepared for the topical application from the following ingredients:

| % Weight | Ingredient |
|---|---|
| 0.06% | Co-dergocrine mesylate |
| 0.3% | Isosorbide dinitrate |
| 2% | aminophylline |

Along with inert carriers of the kind usually used for the preparation of creams.

### Example 3 :

### Preparation C: Cream preparation for topical use:

A cream was prepared for topical application, from the following ingredients

| %Weight | Ingredient |
|---|---|
| 0.06% | Co-dergocrine mesylate |
| 0.4% | Isosorbide dinitrate |

### Example 4

### Preparation D: Cream preparation for topical use:

A cream was prepared for topical application, from the following ingredients

| %Weight | Ingredients |
|---|---|
| 0.06% | Co-dergocrine mesylate |
| 0.3% | Isosorbide dinitrate |
| 2% | Thiophylline |

The action of these preparations on the increment of mean blood flow velocity of cavernous artery, for comparison purposes. Table 5 shows the results.

**Table 5:**

| Mean blood velocity ( M/S) by the application of the present invention preparations: | | | | |
|---|---|---|---|---|
| | FORMULA (A) | FORMULA (B) | FORMULA(C) | FORMULA(D) |
| 1 | 0.26 | 0.27 | 0.25 | 0.29 |
| 2 | 0.28 | 0.28 | 0.29 | 0.3 |
| 3 | 0.23 | 022 | 0.21 | 0.32 |
| 4 | 0.28 | 0.28 | 0.23 | 0.26 |
| 5 | 0.25 | 0.29 | 0.24 | 0.28 |

From the previous experiments and examples, it appears that the invention compositions were effective 3 times more that each ingredient separately.

Meanwhile they have no serious side effects as the case with the injections used in the past.

On the other hand clinical studies have been effected to determine the action of this composition and its capacity in the treatment of erectile dysfunction caused by various factors.

The results of this study are shown in Table 6.

**Table 6:**

| Penile response after topical application of the invention compositions | | | | | |
|---|---|---|---|---|---|
| Etiology | No. of patients | RESPONSE/NO. OF RESPONDERS | | | |
| | | Full | Partial | Tumersence | NO |
| Psychogenic | 9 | 8 | | | 1 |
| Neurogenic | 8 | 4 | 1 | 1 | 2 |
| Arterial insufficiency | 7 | 2 | 1 | 1 | 3 |
| Venous leakage | 4 | 1 | | | 3 |
| others | 8 | 6 | | | 2 |
| TOTAL | 36 | 21 | 2 | 2 | 11 |

These results show that the present composition is capable of treating 58% in patients of erectile dysfunction, by the application of 2 gm of the preparation, daily for a period of one month.

The preparation has shown its highest action, which is 89%, in the treatment of erectile dysfunction in Psychogenic patients.

Since the preparation is in the form of topical cream, gel or spray, it has no systemic side effects.

In a group of other trials, testosterone was added to the inventive preparations to compensate cases suffer from deficiency of hormones in old aged, or due to disease reasons.

These results are shown in the following table.

### Table 7

Penile response after topical application of the inventive composition + hormone:

**TABLE 7**

| Etiology | No. of Patients | RESPONSE/NO. OF RESPONDERS | | | |
|---|---|---|---|---|---|
| | | Full | Partial | Tumersence | NO |
| Psychogenic | 8 | 8 | | | |
| Neurogenic | 5 | 2 | | | 2 |
| Arterial Insufficiency | 8 | 4 | | | 3 |
| Venous leakage | 5 | 2 | 1 | | 2 |
| others | 10 | 7 | 1 | | 2 |
| TOTAL | 36 | 23 | 2 | 2 | 9 |

These results show the Testosterone hormone has increased the action to 64% as Testosterone has effective ability to improve the desire which improvestheir response to the treatment with the composition of the present invention.

## Claims

1. A composition for use in the treatment of erectile dysfunction comprising a mixture ofthe following ingredients:
a- Theophylline or aminophylline
b- Isosorbide dinitrate
c- Co-dergocrine mesylate (dihydro argotoxine mesylate) along with normal carriers and catalysts.

2. A composition for the treatment of erectile dysfunction according to claim 1, wherein Theophylline or aminophylline is used in the range from 0.1% to 6% by weight, and preferably from 1% to 4% by weight.

3. A composition for the treatment of erectile dysfunction according to claim 1 wherein isosorbide dinitrate is used in the range from 0.1% to 0.8%, and preferably from 0.1% to 0.5% by weight.

4. A composition for the treatment of erectile dysfunction according to claim 1 wherein Co-dergocrine mesylate is used in the range from 0.02% to 0.1%, and preferably from 0.04 to 0.08% by weight.

5. A composition according to any preceding claim which composition further comprises Testosterone.

6. A composition according to any preceding claim in the form of cream, ointment gel, normal spray or under pressure spray.

7. Use of a composition in the preparation of a medicament for the treatment of erectile dysfunction (in men) which composition comprises a mixture of the following ingredients:
a- Theophylline or aminophylline
b- Isosorbide dinitrate
c- Co-dergocrine mesylate (dihydro argotoxine mesylate)

8. Use according to claim 7 wherein theophylline or aminophylline is in the range from 0.1% to 6% by weight, and preferably from 1% to 4%.

9. Use according to claim 7, wherein isosorbide dinitrate is used in a range from 0.01% - 0.8% and preferably from 0.1% - 0.5% by weight.

10. Use according to claim 7 wherein Co-dergocrine mesylate is used in a quantity from 0.05% - 0.1% preferably from 0.04-0.08% by weight.

11. Use of theophylline or aminophylline ingredients in the treatment of erectile dysfunction.

12. Use of isosorbide dinitrate in treating erectile dysfunction.

13. Use of Co-dergocrine mesylate in the treatment of erectile dysfunction.
